# EUROPEAN PATENT APPLICATION

(11) **EP 4 170 557 A1**
(43) Date of publication of application: **26.04.2023**
(21) Application number: 22190746.2
(22) Date of filing: 17.08.2022
(51) Int. Cl.: G06N 5/045, G06Q 10/105, G06N 3/0442, G06N 3/0464, G06N 3/098

(54) **MACHINE LEARNING BASED PERFORMANCE PREDICTION**

(30) Priority: 22.10.2021 US 202117508699
(71) Applicant: SAP SE, 69190 Walldorf (DE)
(72) Inventor: ANAMANDRA, Sai Hareesh, 69190 Walldorf (DE); KRISHNAN, Kavitha, 69190 Walldorf (DE); JALAGADUGULA, Rohit, 69190 Walldorf (DE); MENON, Parthasarathy, 69190 Walldorf (DE); D'SOUZA, Aditi, 69190 Walldorf (DE); MOHANTY, Shrusti, 69190 Walldorf (DE); BU, Lingyun, 69190 Walldorf (DE); ROY, Vinay George, 69190 Walldorf (DE)
(74) Representative: Müller-Boré & Partner Patentanwälte PartG mbB

(57) **Abstract**

A method may include training one or more machine learning models to predict a decline in employee performance. The machine learning models may be trained in a federated manner to avoid the exchange of personal data. The trained machine learning models may be applied to data associated with an employee that corresponds to one or more leading indicators of employee burnout. In response to the trained machine learning models predicting a decline in the performance of the employee, the root causes of the predicted decline in the performance of the employee may be identified by applying an explainability algorithm such as Shapley Additive Explanations (SHAP). A report including a corrective action for the predicted decline in employee performance may be generated based on the root causes. Related systems and computer program products are also provided.

## Description

### TECHNICAL FIELD

The subject matter described herein relates generally to a machine learning and more specifically to a machine learning based performance prediction.

### SUMMARY

Systems, methods, and articles of manufacture, including computer program products, are provided for a machine learning based performance prediction. In one aspect, there is provided a system. The system may include at least one data processor and at least one memory. The at least one memory may store instructions that cause operations when executed by the at least one data processor. The operations may include: training one or more machine learning models to predict a decline in employee performance; applying, to a data associated with an employee, the one or more trained machine learning models to predict a decline in a performance of the employee; in response to the one or more trained machine learning models predicting the decline in the performance of the employee, determining one or more root causes of the predicted decline in the performance of the employee; and generating, based at least on the one or more root causes, a report including a corrective action for the predicted decline in the performance of the employee.

In some variations, one or more features disclosed herein including the following features can optionally be included in any feasible combination. The one or more root causes may be identified by applying an explainability algorithm to determine a top k quantity of variables contributing to an output of the one or more machine learning models.

In some variations, the explainability algorithm may include a Shapley Additive Explanations (SHAP).

In some variations, the one or more machine learning models may include a convolutional neural network, a recurrent neural network, a regression model, an instance-based model, a regularization model, a decision tree, a random forest, a Bayesian model, a clustering model, an associative model, a deep learning model, a dimensionality reduction model, and/or an ensemble model.

In some variations, the training of the one or more machine learning models may include training, at a first client device associated with the employee, a first local machine learning model, the local machine learning model being trained based at least on a first personal data associated with the employee, and updating, based at least on a first parameter space of the trained first local machine learning model, a second parameter space of a global machine learning model deployed at a server.

In some variations, the training of the one or more machine learning models may further include updating, based at least on a third parameter space of a second local machine learning model, the second parameter space of the global machine learning model, the second machine learning model being deployed at a second client device of another employee, and the second machine learning model being trained based on a second personal data of the another employee, and updating, based at least on the updated second parameter space of the global machine learning model, the first parameter space of the first local machine learning model and the third parameter space of the second local machine learning model.

In some variations, the operations may further include: preprocessing at least a portion of the data associated with the employee, the preprocessing includes performing a sentiment analysis to determine a type of sentiment, a fatigue level, an alertness level, and/or an engagement level exhibited by the employee.

In some variations, the data associated with the employee may correspond to one or more leading indicators of employee burnout.

In some variations, the data associated with the employee may include a physical activity pattern, a heartbeat pattern, a meal pattern, and/or a sleep pattern of the employee.

In some variations, the data associated with the employee may include a quantity of time spent interacting with different types of applications installed on an edge device of the employee.

In some variations, the data associated with the employee may include a quantity of deadlines, an urgency of deadlines, and/or a delay in meeting deadlines.

In some variations, the data associated with the employee may include one or more key events, personal milestones, professional milestones, holidays, seasonal constraints, and job role constraints.

In another aspect, there is provided a method for a machine learning based performance prediction. The method may include: training one or more machine learning models to predict a decline in employee performance; applying, to a data associated with an employee, the one or more trained machine learning models to predict a decline in a performance of the employee; in response to the one or more trained machine learning models predicting the decline in the performance of the employee, determining one or more root causes of the predicted decline in the performance of the employee; and generating, based at least on the one or more root causes, a report including a corrective action for the predicted decline in the performance of the employee.

In some variations, one or more features disclosed herein including the following features can optionally be included in any feasible combination. The one or more root causes may be identified by applying an explainability algorithm to determine a top k quantity of variables contributing to an output of the one or more machine learning models.

In some variations, the training of the one or more machine learning models may include training, at a first client device associated with the employee, a first local machine learning model, the local machine learning model being trained based at least on a first personal data associated with the employee, updating, based at least on a first parameter space of the trained first local machine learning model, a second parameter space of a global machine learning model deployed at a server, updating, based at least on a third parameter space of a second local machine learning model, the second parameter space of the global machine learning model, the second machine learning model being deployed at a second client device of another employee, and the second machine learning model being trained based on a second personal data of the another employee, and updating, based at least on the updated second parameter space of the global machine learning model, the first parameter space of the first local machine learning model and the third parameter space of the second local machine learning model.

In some variations, the data associated with the employee may include a physical activity pattern, a heartbeat pattern, a meal pattern, and/or a sleep pattern of the employee.

In some variations, the data associated with the employee may include a quantity of time spent interacting with different types of applications installed on an edge device of the employee.

In some variations, the data associated with the employee may include a quantity of deadlines, an urgency of deadlines, and/or a delay in meeting deadlines.

In some variations, the data associated with the employee may include one or more key events, personal milestones, professional milestones, holidays, seasonal constraints, and job role constraints.

In another aspect, there is provided a computer program product that includes a non-transitory computer readable storage medium. The non-transitory computer-readable storage medium may include program code that causes operations when executed by at least one data processor. The operations may include: training one or more machine learning models to predict a decline in employee performance; applying, to a data associated with an employee, the one or more trained machine learning models to predict a decline in a performance of the employee; in response to the one or more trained machine learning models predicting the decline in the performance of the employee, determining one or more root causes of the predicted decline in the performance of the employee; and generating, based at least on the one or more root causes, a report including a corrective action for the predicted decline in the performance of the employee.

Implementations of the current subject matter can include, but are not limited to, methods consistent with the descriptions provided herein as well as articles that comprise a tangibly embodied machine-readable medium operable to cause one or more machines (e.g., computers, etc.) to result in operations implementing one or more of the described features. Similarly, computer systems are also described that may include one or more processors and one or more memories coupled to the one or more processors. A memory, which can include a non-transitory computer-readable or machine-readable storage medium, may include, encode, store, or the like one or more programs that cause one or more processors to perform one or more of the operations described herein. Computer implemented methods consistent with one or more implementations of the current subject matter can be implemented by one or more data processors residing in a single computing system or multiple computing systems. Such multiple computing systems can be connected and can exchange data and/or commands or other instructions or the like via one or more connections, including, for example, to a connection over a network (e.g. the Internet, a wireless wide area network, a local area network, a wide area network, a wired network, or the like), via a direct connection between one or more of the multiple computing systems, etc.

The details of one or more variations of the subject matter described herein are set forth in the accompanying drawings and the description below. Other features and advantages of the subject matter described herein will be apparent from the description and drawings, and from the claims. While certain features of the currently disclosed subject matter are described for illustrative purposes in relation to a machine learning based performance prediction, it should be readily understood that such features are not intended to be limiting. The claims that follow this disclosure are intended to define the scope of the protected subject matter.

### DESCRIPTION OF DRAWINGS

The accompanying drawings, which are incorporated in and constitute a part of this specification, show certain aspects of the subject matter disclosed herein and, together with the description, help explain some of the principles associated with the disclosed implementations. In the drawings,
FIG. 1 depicts a system diagram illustrating an example of a diagnostic system, in accordance with some example embodiments;
FIG. 2 depicts a block diagram illustrating an example data architecture for machine learning based performance prediction, in accordance with some example embodiments;
FIG. 3A depicts a block diagram illustrating an example of data aggregation and anonymization, in accordance with some example embodiments;
FIG. 3B depicts a block diagram illustrating another example of data aggregation and anonymization, in accordance with some example embodiments;
FIG. 3C depicts a block diagram illustrating another example of data aggregation and anonymization, in accordance with some example embodiments;
FIG. 3D depicts a block diagram illustrating another example of data aggregation and anonymization, in accordance with some example embodiments;
FIG. 3E depicts a block diagram illustrating another example of data aggregation and anonymization, in accordance with some example embodiments;
FIG. 3F depicts a block diagram illustrating an example of an edge device, in accordance with some example embodiments;
FIG. 4 depicts a schematic diagram illustrating an example of federated learning, in accordance with some example embodiments;
FIG. 5 depicts a flowchart illustrating an example of a process for machine learning based performance prediction, in accordance with some example embodiments;
FIG. 6 depicts a flowchart illustrating an example of a process for federated learning, in accordance with some example embodiments;
FIG. 7 depicts a block diagram illustrating an example of a computing system, in accordance with some example embodiments.

When practical, similar reference numbers denote similar structures, features, or elements.

### DETAILED DESCRIPTION

Optimal employee performance is crucial an organization's success but personal and professional stressors can cause employee burnout, which may manifest in a variety of detrimental symptoms such as exhaustion, cynicism, and diminished professional efficacy. An organization may realize tremendous benefits by detecting employee burnout as early as possible. However, conventional approaches, which rely on lagging indicators that are present when burnout has already developed, are reactive in nature and less effective at avoiding the consequences of burnout altogether. As such, in some example embodiments, a diagnostic engine may apply one or more machine learning models trained to predict declines in employee performance. The trained machine learning models may be applied to a variety of data corresponding to the leading indicators of employee burnout including, for example, productivity data, personal data, human resource data, information technology data, and contextual data. In doing so, the diagnostic engine may be capable of detecting early signs of burnout and predict the concomitant decline in employee performance ahead of time such that proactive measures may be taken to avoid actual declines in employee performance.

At least a portion of the data used to predict performance may be private data that should remain confidential. Accordingly, in some example embodiments, the training of the one or more machine learning models may include federated learning (or collaborative learning) in which a global machine learning model is trained collaboratively through training local machine learning models across multiple, decentralized client devices. For example, a first local machine learning model may be trained, at a first client device associated with a first employee, using a first personal data of the first employee while a second local machine learning model may be trained, at a second client device associated with a second employee, using a second personal data of the second employee. The federated learning approach may train the global machine learning model without an exchange of the personal data. For instance, instead of the global machine learning model being trained directly using the first personal data and the second personal data, the training of the global machine learning model may include updating the parameter space of the global machine learning model with those the trained first local machine learning model and the trained second local machine learning model. The updated parameter space of the global machine learning model may subsequently be propagated to each of the first local machine learning model at the first client device and the second local machine learning model at the second client device.

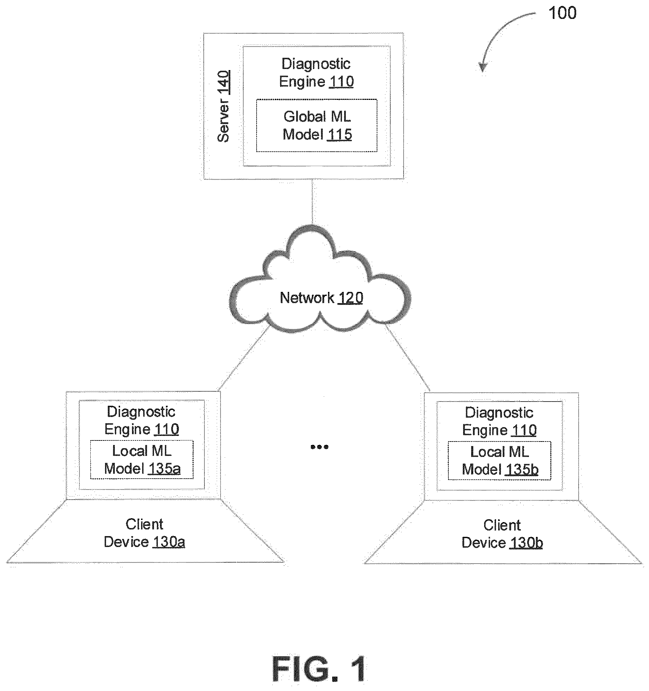

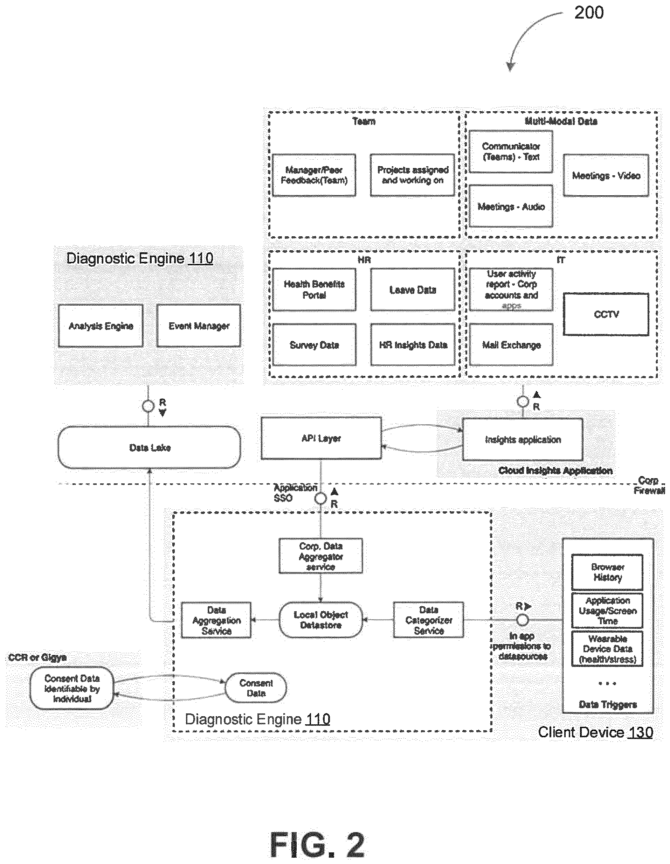

FIG. 1 depicts a system diagram illustrating an example of a diagnostic system 100, in accordance with some example embodiments. An example of the data architecture implemented by the diagnostic system 100 is shown in FIG. 2. Referring to FIGS. 1-2, the diagnostic system 100 may include a diagnostic engine 110 that is communicatively coupled, via a network 120, to one or more client devices 130 including, for example, a first client device 130a, a second client device 130b, and/or the like. The network 120 may be any wired and/or wireless network including, for example, a public land mobile network (PLMN), a wide area network (WAN), a local area network (LAN), a virtual local area network (VLAN), the Internet, and/or the like. The one or more client devices 130 may be processor-based devices including, for example, smartphones, tablet computers, wearable apparatuses, virtual assistants, Internet-of-Things (IoT) appliances, and/or the like.

In some example embodiments, the diagnostic engine 110 may apply one or more machine learning models trained to predict declines in employee performance. Examples of machine learning models may include a convolutional neural network, a recurrent neural network, a regression model, an instance-based model, a regularization model, a decision tree, a random forest, a Bayesian model, a clustering model, an associative model, a deep learning model, a dimensionality reduction model, and/or an ensemble model. In some cases, the diagnostic engine 110 may apply a first machine learning model trained to predict a decline in employee performance and a second machine learning model trained to determine one or more root causes for the predicted decline in employee performance. For example, the first machine learning model may be a recurrent neural network (e.g., a long short term memory (LSTM) network and/or the like) trained to detect, in a sequence of measurements corresponding to the leading indicators of burnout, trends indicative of an impending decline in employee performance. Meanwhile, the second machine learning model may be an anomaly detection algorithm (e.g., a clustering model, a Hidden Markov model, a Bayesian network, and/or the like) trained to detect, in the measurements corresponding to the leading indicators of burnout, one or more outlier values indicative of a root cause of the impending decline in employee performance.

The trained machine learning models may be applied to a variety of data corresponding to the leading indicators of employee burnout including, for example, productivity data, personal data, human resource data, information technology data, and contextual data. In doing so, the diagnostic engine 110 may be capable of detecting early signs of burnout and predict the concomitant decline in employee performance ahead of time such that proactive measures may be taken to avoid actual declines in employee performance. Examples of productivity data may include workload (e.g., meetings, deadlines, work life-balance, and/or the like), engagement level, peer feedback, collaborative engagements, quantity of productive time, quantity and urgency of deadlines, and delays in meeting deadlines. Examples of personal data may include social situation, family situation, level of physical activity, quantity of personal time, sleep patterns, meal patterns, health patterns, and environmental factors. Examples of human resource data may include leave patterns, compensation, workload, and performance evaluations. Examples of contextual data may include the occurrence of key events, personal and/or professional milestones, holidays, seasonal constraints, job role constraints and attributes, achievements, health, current context of the employee, and team composition.

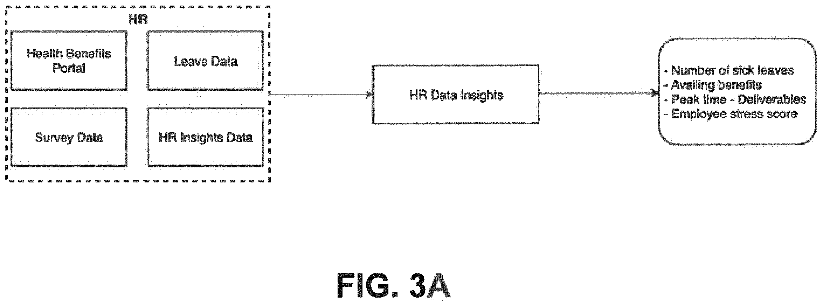

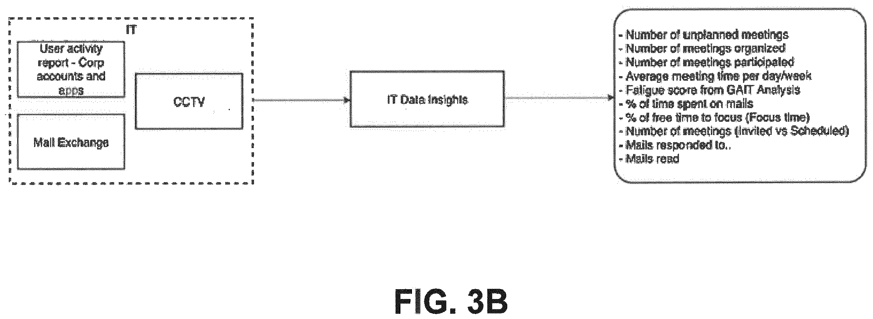

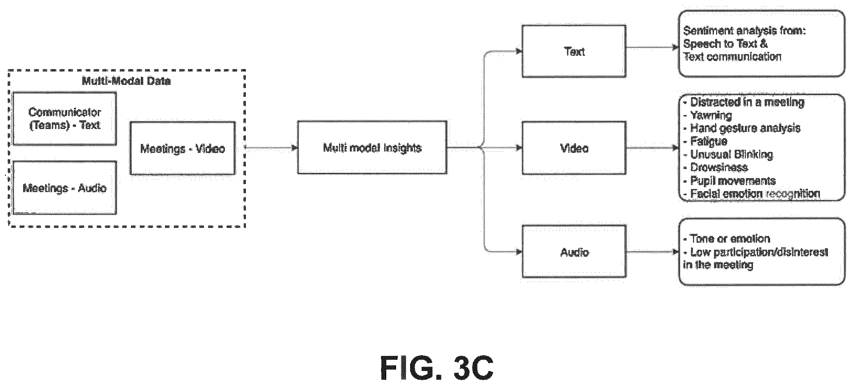

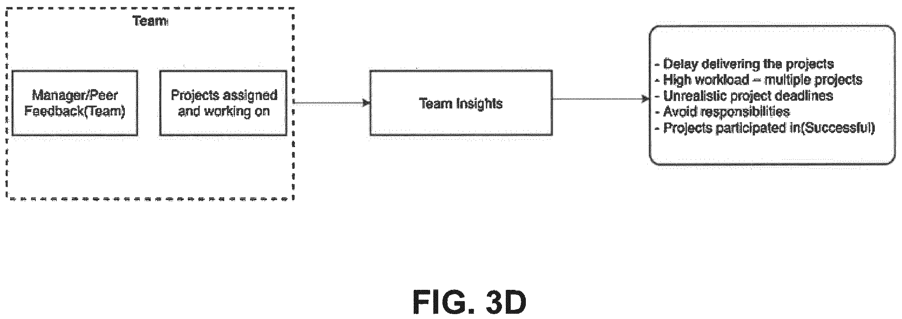

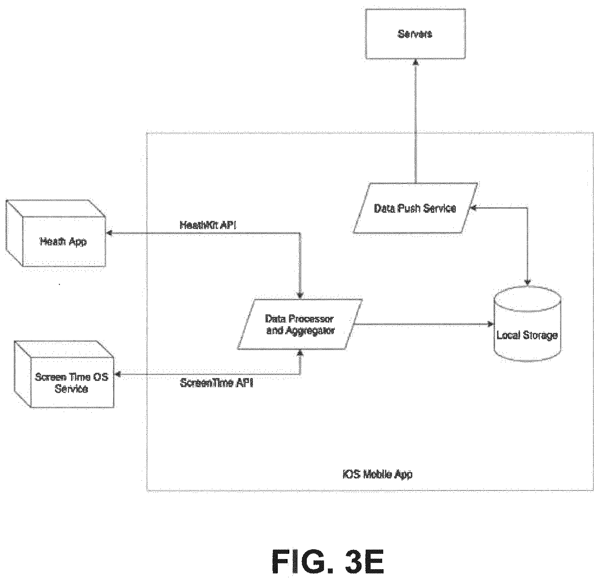

At least a portion of the data used to predict performance may be private data that should remain confidential. As such, the diagnostic engine 110 may anonymize data collected from a variety of sources. FIGS. 3A-E depict block diagrams illustrating various examples of data aggregation and preprocessing including anonymization, which the diagnostic engine 110 may perform in order to aggregate, from various sources, data corresponding to the leading indicators of employee burnout such as productivity data, personal data, human resource data, information technology data, and contextual data. Referring to FIG. 3E, one example of data that may be aggregated for analysis by the trained machine learning models include multi-modal data such as text data from communication applications (e.g., instant messaging applications, short messaging service (SMS) applications, and/or the like), audio data (e.g., from meetings), and video data (e.g., from meetings). In some cases, multi-modal data may be preprocessed before being analyzed by the trained machine learning models. For example, text data, which may include transcribed speech data from the audio and/or video data, may undergo sentiment analysis to flag the text data as being associated with one or more positive sentiments, negative sentiments, or neutral sentiments. Audio data may be analyzed for tone of the voice, which in turn may be used to determine a fatigue level, an alertness level, an engagement level, and/or other emotions exhibited by the employees associated with the audio data. The video data may also be analyzed for physical indicators (e.g., pupil movement and/or the like) of fatigue, alertness, engagement, and/or other emotions such as happiness, excitement, fear, anger, and disgust.

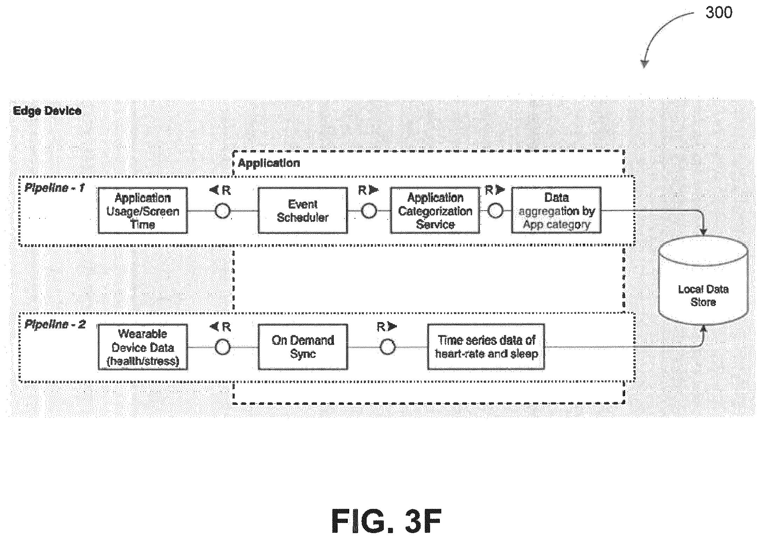

At least a portion of the leading indicators of employee burnout may originate from edge devices such as, for example, the first client device 130a, the second client device 130b, and/or the like. FIG. 3F depicts a block diagram illustrating an example of an edge device 300, in accordance with some example embodiments. As shown in FIGS. 3E-F, the edge device 300 may track screen time, which may be further categorized into quantities of time spent interacting with different types of applications installed on the edge device such as, for example, productivity applications, social media applications, educational applications, and/or the like. Screen time data that has been categorized in accordance with the type of application may provide insight how time utilization and management. Alternatively and/or additionally, the edge device 300 may track health data, which may provide insights into punctuality, regularity of the physical activity patterns, heartbeat patterns, meal patterns, and sleep patterns. One or more changes in health patterns may correlate with behavioral changes and indicate a potential source for stress and anxiety.

In some example embodiments, data privacy may be further maximized by applying a federated learning approach to minimize the exchange of private data. To further illustrate, FIG. 1 shows that the diagnostic engine 110 including a global machine learning model 115 may be deployed at a server 140 while each of the client devices 130 may host the diagnostic engine 110 including a respective local machine learning model 135 such as, for example, a first local machine learning model 135a at the first client device 130a, a second local machine learning model 135b at the second client device 130b, and/or the like. The first local machine learning model 135a may be trained based on a first private data of a first employee associated with the first client device 130a and the second local machine learning model 135b may be trained based on a second private data of a second employee associated with the second client device 130b. For example, the training data applied to train the first local machine learning model 135a and the second machine learning model 135b may include a first data corresponding to one or more leading indicators of employee burnout and a second data corresponding to one or more lagging indicators of employee burnout. Examples of lagging indicators may include lack of motivation, missed deadlines, frequent leaves, poor health, emotional outbursts, excessive pressure on keyboard, attribution, tardiness, and lack of hobbies. The lagging indicators may serve as ground truth labels for a combination of leading indicators that are indicative of impending employee burnout. Thus, the training of the first local machine learning model 135a and the second machine learning model 135b may include adjusting the weights applied by each model when processing the first data such that a difference between the output of the models and the ground truths labels included in the second data is minimized.

With federated learning, instead of the global machine learning model 115 being trained directly using the first personal data and the second personal data, the training of the global machine learning model 115 may include updating the parameter space of the global machine learning model 115 with those the trained first local machine learning model 135a and the trained second local machine learning model 135b. The updated parameter space of the global machine learning model 115 may subsequently be propagated to each of the first local machine learning model 135a at the first client device 130a and the second local machine learning model 135b at the second client device 130b. From a tractability standpoint, the quantity of local machine learning models, such as the first local machine learning model 135a and the second machine learning model 135b, may be limited by creating role-specific local machine learning models instead of user-specific local machine learning models.

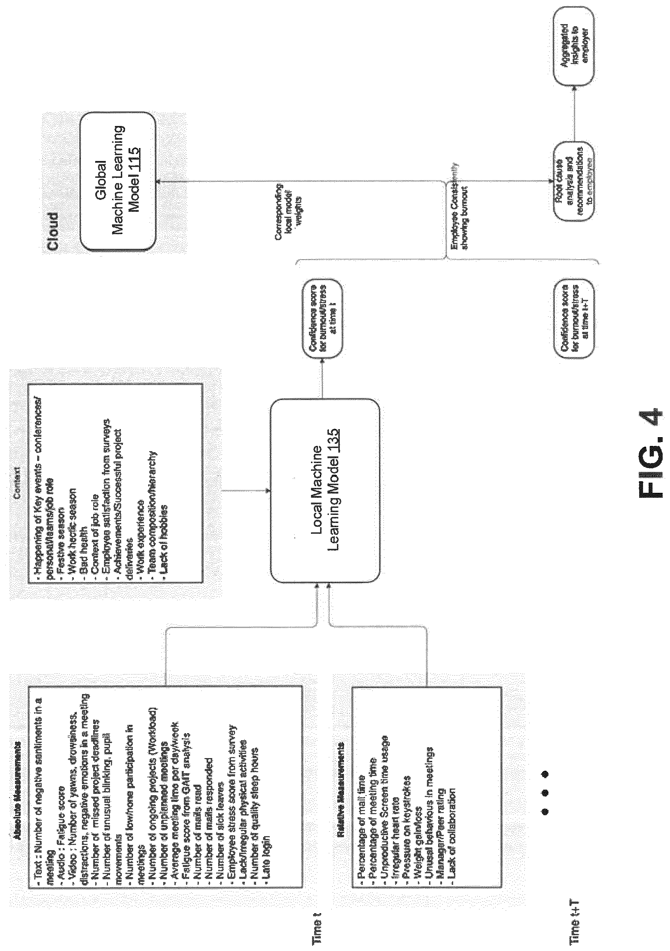

FIG. 4 depicts a schematic diagram illustrating an example of federated learning, in accordance with some example embodiments. As shown in FIG. 4, at least a portion of the data corresponding to the leading indicators of the employee burnout be time series data that include a sequence of absolute measurement values and/or relative measurement values. Absolute measurements may include leading indicators that can be ingested directly by the local machine learning model 135, which may be deployed at the client device 130 associated with a particular employee. Meanwhile, relative measurements may include leading indicators that are calculated relative to a norm value for a single employee or a group of similar employees (e.g., an mean, a median, a mode, and/or the like). In addition to the absolute measurements and relative measurements, the local machine learning model 135 may also ingest context data, which may include metadata associated with the employee.

As shown in FIG. 4, the local machine learning model 135 may be trained to determined, based at least on the absolute measurements, the relative measurements, and the context data, an output value corresponding to a prediction of employee performance. For example, the local machine learning model 135 may output a confidence score indicative of whether the employee is likely to experience burnout at various points in time (e.g., at time *t*, *t*+T, and/or the like). In the event the output of the local machine learning model 135 indicates the employee as exhibiting signs of burnout more than a threshold quantity of time, a root cause analysis may be performed to determine, for example, a top k quantity of root causes for the burnout and corrective actions addressing these root causes. Moreover, information associated with at least a portion of employees, such as the results of performance prediction and root cause analysis, may be aggregated and shared with an employer.

The local machine learning model 135 may be configured to perform its inference at regular intervals with adjustable granularity (e.g., hourly, weekly, monthly, and/or the like). In some cases, the training data for the local machine learning model 135 may include data from earlier points in time, with the pervious output of the local machine learning model 135 (e.g., the confidence score indicative of the likelihood of burnout) being used as labels for the corresponding data. With the federated learning approach, the global machine learning model 115 may be trained by updating the parameter space of the global machine learning model 115 with the parameter space of the trained local machine learning model 135. In the example shown in FIG. 4, the training of the global machine learning model 115 may include propagating the weights applied at the local machine learning model 135 to the global machine learning machine model 115 such that the weights of the global machine learning model 115 may be updated accordingly. Doing so may train the global machine learning model 115 to include the insights learned at the local machine learning model 135 without sharing the absolute measurements, relative measurements, and context data available at the client device 130.

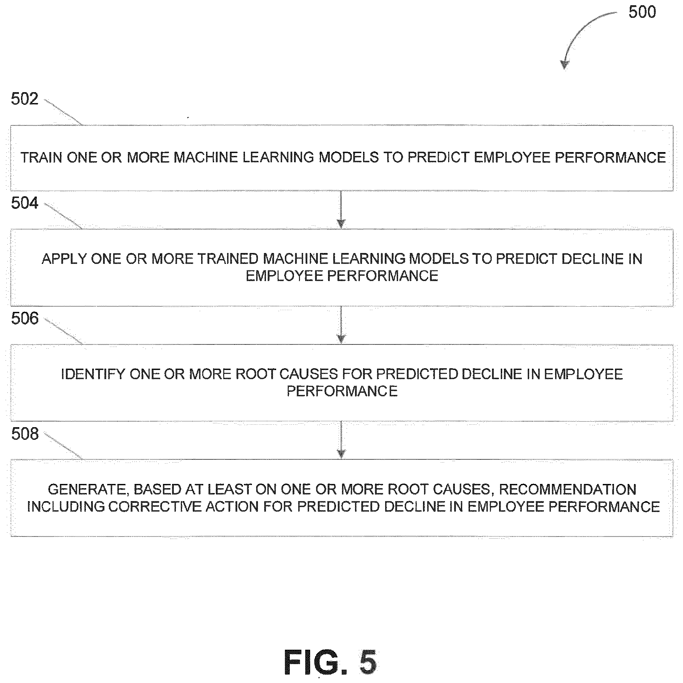

FIG. 5 depicts a flowchart illustrating an example of a process 500 for machine learning based performance prediction, in accordance with some example embodiments. Referring to FIG. 5, the process 500 may be performed by the diagnostic engine 110.

At 502, the diagnostic engine 110 may train one or more machine learning models to predict employee performance. In some example embodiments, the diagnostic engine 110 may train one or more machine learning models to predict declines in employee performance. For example, the one or more machine learning models may be trained to detect, based on data corresponding to one or more leading indicators of burnout, early signs of burnout. Examples of machine learning models may include a convolutional neural network, a recurrent neural network, a regression model, an instance-based model, a regularization model, a decision tree, a random forest, a Bayesian model, a clustering model, an associative model, a deep learning model, a dimensionality reduction model, and/or an ensemble model. Data corresponding to the leading indicators of burnout may include productivity data, personal data, human resource data, information technology data, and contextual data.

In some cases, a first machine learning model may be trained to predict a decline in employee performance while a second machine learning model may be trained to determine one or more root causes for the predicted decline in employee performance. For example, the first machine learning model may be a recurrent neural network (e.g., a long short term memory (LSTM) network and/or the like) trained to detect, in a sequence of measurements corresponding to the leading indicators of burnout, trends indicative of an impending decline in employee performance. Meanwhile, the second machine learning model may be an anomaly detection algorithm (e.g., a clustering model, a Hidden Markov model, a Bayesian network, and/or the like) trained to detect, in the measurements corresponding to the leading indicators of burnout, one or more outlier values indicative of a root cause of the impending decline in employee performance.

At 504, the diagnostic engine 110 may apply the one or more trained machine learning models to predict a decline in employee performance. The trained machine learning models may be applied to a variety of data corresponding to the leading indicators of employee burnout including, for example, productivity data, personal data, human resource data, information technology data, and contextual data. By applying the machine learning models to detect early signs of burnout, the diagnostic engine 110 may be able to predict the concomitant decline in employee performance ahead of time such that proactive measures may be taken to avoid actual declines in employee performance.

At 506, the diagnostic engine 110 may determine one or more root causes of the predicted decline in employee performance. For example, in the event the output of the machine learning models predicts a decline in employee performance for example, as a result of impending burnout, the diagnostic engine 110 may determine a top k quantity of root causes for the predicted decline. As noted, root cause determination may also be performed by applying a trained machine learning model such as, for example, an anomaly detection algorithm (e.g., a clustering model, a Hidden Markov model, a Bayesian network, and/or the like) trained to detect, in the measurements corresponding to the leading indicators of burnout, one or more outlier values corresponding to the root causes of the burnout. Alternatively and/or additionally, root cause determination may be performed by applying an interpretability or explainability algorithm, such as Shapley Additive Explanations (SHAP), to determine the top k variables contributing to the output of the machine learning models predicting a decline in employee performance. This k quantity of variables may be identified as the root causes of the predicted decline in employee performance.

At 508, the diagnostic engine 110 may generate, based at least on the one or more root causes, a report including a corrective action for the predicted decline in employee performance. For example, the diagnostic engine 110 may determine corrective actions for alleviating and/or eliminating the root causes of the predicted decline in employee performance. In cases where the corrective actions include actions performed by the employee as well as the employer, this information may be aggregated and shared with the employee and the employer.

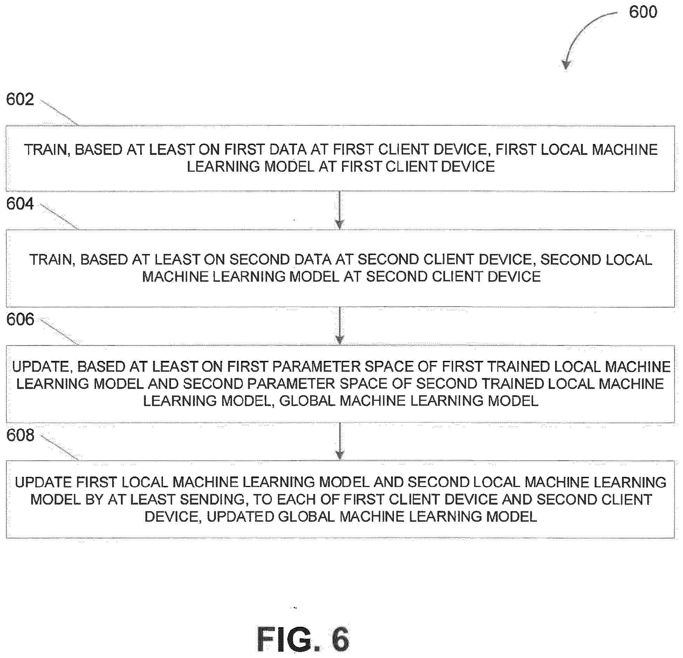

FIG. 6 depicts a flowchart illustrating an example of a process 600 for federated learning, in accordance with some example embodiments. Referring to FIG. 6, the process 500 may be performed by the diagnostic engine 110 and may implement, for example, operation 502 of the process 500.

At 602, the diagnostic engine 110 may train, based at least on a first data at a first client device, a first local machine learning model at the first client device. For example, the diagnostic engine 110 deployed at the first client device 130a may train, based on a first private data of a first employee associated with the first client device 130a, the first local machine learning model 135a at the first client device 130a.

At 604, the diagnostic engine 110 may train, based at least on a second data at a second client device, a second local machine learning model at the second client device. For example, the second local machine learning model 135b at the second client device 130b may be trained based at least on a second private data of a second employee associated with the second client device 130b.

At 606, the diagnostic engine 110 may update, based at least on a first parameter space of the first trained local machine learning model and/or a second parameter space of the second trained local machine learning model, a global machine learning model. In some example embodiments, instead of training the global machine learning model 115 with data from the first client device 130a and the second client device 130b, the global machine learning model 115 may be trained by updating the parameter space of the global machine learning model 115 with those of the trained first local machine learning model 135a and the second local machine learning model 135b. For example, the training of the global machine learning model 115 may include propagating the weights applied at the first local machine learning model 135a and the second local machine learning model 135b to the global machine learning machine model 115 such that the weights of the global machine learning model 115 may be updated accordingly. Doing so may train the global machine learning model 115 to include the insights learned at the first local machine learning model 135a and the second local machine learning model 135b without sharing private data from the first client device 130a and the second client device 130b.

At 608, the diagnostic engine 110 may update the first local machine learning model and the second local machine learning model by at least sending, to each of the first client device and the second client device, the updated global machine learning model. In some example embodiments, the updated global machine learning model 115 may be propagated to each of the first client device 130a and the second client device 130b such that the first local machine learning model 135a and the second local machine learning model 135b may be updated accordingly. For example, the parameter space of the global machine learning model 115, which has been updated based on those of the first local machine learning model 135a and the second local machine learning model 135b, may be used to update the parameter spaces of the first local machine learning model 135a and the second local machine learning model 135b. Doing so may allow an exchange of insights learned by each of the first local machine learning model 135a and the second local machine learning model 135b from the locally available private data without an actual exchange of the private data between the first client device 130a and the second client device 130b.

In view of the above-described implementations of subject matter this application discloses the following list of examples, wherein one feature of an example in isolation or more than one feature of said example taken in combination and, optionally, in combination with one or more features of one or more further examples are further examples also falling within the disclosure of this application:
Example 1: A system, comprising: at least one data processor; and at least one memory storing instructions, which when executed by the at least one data processor, result in operations comprising: training one or more machine learning models to predict a decline in employee performance; applying, to a data associated with an employee, the one or more trained machine learning models to predict a decline in a performance of the employee; in response to the one or more trained machine learning models predicting the decline in the performance of the employee, determining one or more root causes of the predicted decline in the performance of the employee; and generating, based at least on the one or more root causes, a report including a corrective action for the predicted decline in the performance of the employee.
Example 2: The system of example 1, wherein the one or more root causes are identified by applying an explainability algorithm to determine a top k quantity of variables contributing to an output of the one or more machine learning models.
Example 3: The system of example 2, wherein the explainability algorithm comprises a Shapley Additive Explanations (SHAP).
Example 4: The system of any one of examples 1 to 3, wherein the one or more machine learning models include a convolutional neural network, a recurrent neural network, a regression model, an instance-based model, a regularization model, a decision tree, a random forest, a Bayesian model, a clustering model, an associative model, a deep learning model, a dimensionality reduction model, and/or an ensemble model.
Example 5: The system of any one of examples 1 to 4, wherein the training of the one or more machine learning models include training, at a first client device associated with the employee, a first local machine learning model, the local machine learning model being trained based at least on a first personal data associated with the employee, and updating, based at least on a first parameter space of the trained first local machine learning model, a second parameter space of a global machine learning model deployed at a server.
Example 6: The system of example 5, wherein the training of the one or more machine learning models further include updating, based at least on a third parameter space of a second local machine learning model, the second parameter space of the global machine learning model, the second machine learning model being deployed at a second client device of another employee, and the second machine learning model being trained based on a second personal data of the another employee, and updating, based at least on the updated second parameter space of the global machine learning model, the first parameter space of the first local machine learning model and the third parameter space of the second local machine learning model.
Example 7: The system of any one of examples 1 to 6, wherein the operations further comprise: preprocessing at least a portion of the data associated with the employee, the preprocessing includes performing a sentiment analysis to determine a type of sentiment, a fatigue level, an alertness level, and/or an engagement level exhibited by the employee.
Example 8: The system of any one of examples 1 to 7, wherein the data associated with the employee corresponds to one or more leading indicators of employee burnout.
Example 9: The system of any one of examples 1 to 8, wherein the data associated with the employee includes a physical activity pattern, a heartbeat pattern, a meal pattern, and/or a sleep pattern of the employee.
Example 10: The system of any one of examples 1 to 9, wherein the data associated with the employee includes a quantity of time spent interacting with different types of applications installed on an edge device of the employee.
Example 11: The system of any one of examples 1 to 10, wherein the data associated with the employee includes a quantity of deadlines, an urgency of deadlines, and/or a delay in meeting deadlines.
Example 12: The system of any one of examples 1 to 11, wherein the data associated with the employee includes one or more key events, personal milestones, professional milestones, holidays, seasonal constraints, and job role constraints.
Example 13: A method, comprising: training one or more machine learning models to predict a decline in employee performance; applying, to a data associated with an employee, the one or more trained machine learning models to predict a decline in a performance of the employee; in response to the one or more trained machine learning models predicting the decline in the performance of the employee, determining one or more root causes of the predicted decline in the performance of the employee; and generating, based at least on the one or more root causes, a report including a corrective action for the predicted decline in the performance of the employee.
Example 14: The method of example 13, wherein the one or more root causes are identified by applying an explainability algorithm to determine a top k quantity of variables contributing to an output of the one or more machine learning models.
Example 15: The method of any one of examples 13 to 14, wherein the training of the one or more machine learning models include training, at a first client device associated with the employee, a first local machine learning model, the local machine learning model being trained based at least on a first personal data associated with the employee, updating, based at least on a first parameter space of the trained first local machine learning model, a second parameter space of a global machine learning model deployed at a server, updating, based at least on a third parameter space of a second local machine learning model, the second parameter space of the global machine learning model, the second machine learning model being deployed at a second client device of another employee, and the second machine learning model being trained based on a second personal data of the another employee, and updating, based at least on the updated second parameter space of the global machine learning model, the first parameter space of the first local machine learning model and the third parameter space of the second local machine learning model.
Example 16: The method of any one of examples 13 to 15, wherein the data associated with the employee includes a physical activity pattern, a heartbeat pattern, a meal pattern, and/or a sleep pattern of the employee.
Example 17: The method of any one of examples 13 to 16, wherein the data associated with the employee includes a quantity of time spent interacting with different types of applications installed on an edge device of the employee.
Example 18: The method of any one of examples 13 to 17, wherein the data associated with the employee includes a quantity of deadlines, an urgency of deadlines, and/or a delay in meeting deadlines.
Example 19: The method of any one of examples 13 to 18, wherein the data associated with the employee includes one or more key events, personal milestones, professional milestones, holidays, seasonal constraints, and job role constraints.
Example 20: A non-transitory computer readable medium storing instructions, which when executed by at least one data processor, result in operations comprising: training one or more machine learning models to predict a decline in employee performance; applying, to a data associated with an employee, the one or more trained machine learning models to predict a decline in a performance of the employee; in response to the one or more trained machine learning models predicting the decline in the performance of the employee, determining one or more root causes of the predicted decline in the performance of the employee; and generating, based at least on the one or more root causes, a report including a corrective action for the predicted decline in the performance of the employee.

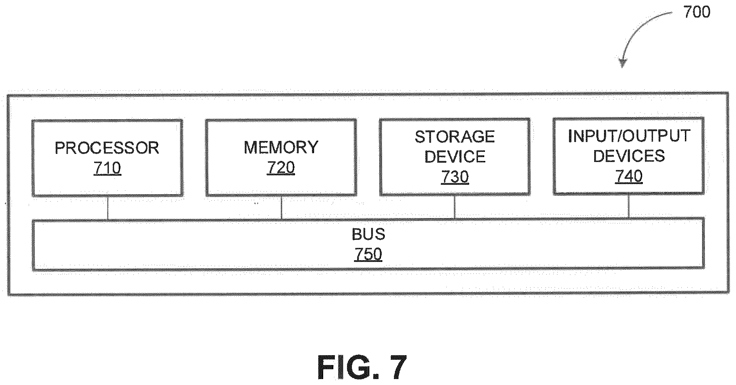

FIG. 7 depicts a block diagram illustrating a computing system 700, in accordance with some example embodiments. Referring to FIGS. 1-7, the computing system 700 can be used to implement the diagnostic engine 110 and/or any components therein.

As shown in FIG. 7, the computing system 700 can include a processor 710, a memory 720, a storage device 730, and input/output devices 740. The processor 710, the memory 720, the storage device 730, and the input/output devices 740 can be interconnected via a system bus 750. The processor 710 is capable of processing instructions for execution within the computing system 700. Such executed instructions can implement one or more components of, for example, the diagnostic engine 110 and/or the like. In some example embodiments, the processor 710 can be a single-threaded processor. Alternately, the processor 710 can be a multi-threaded processor. The processor 710 is capable of processing instructions stored in the memory 720 and/or on the storage device 730 to display graphical information for a user interface provided via the input/output device 740.

The memory 720 is a computer readable medium such as volatile or non-volatile that stores information within the computing system 700. The memory 720 can store data structures representing configuration object databases, for example. The storage device 730 is capable of providing persistent storage for the computing system 700. The storage device 730 can be a floppy disk device, a hard disk device, an optical disk device, or a tape device, or other suitable persistent storage means. The input/output device 740 provides input/output operations for the computing system 700. In some example embodiments, the input/output device 740 includes a keyboard and/or pointing device. In various implementations, the input/output device 740 includes a display unit for displaying graphical user interfaces.

According to some implementations of the current subject matter, the input/output device 740 can provide input/output operations for a network device. For example, the input/output device 740 can include Ethernet ports or other networking ports to communicate with one or more wired and/or wireless networks (e.g., a local area network (LAN), a wide area network (WAN), the Internet).

In some example embodiments, the computing system 700 can be used to execute various interactive computer software applications that can be used for organization, analysis and/or storage of data in various (e.g., tabular) format (e.g., Microsoft Excel^{®}, and/or any other type of software). Alternatively, the computing system 700 can be used to execute any type of software applications. These applications can be used to perform various functionalities, e.g., planning functionalities (e.g., generating, managing, editing of spreadsheet documents, word processing documents, and/or any other objects, etc.), computing functionalities, communications functionalities, etc. The applications can include various add-in functionalities (e.g., SAP Integrated Business Planning add-in for Microsoft Excel as part of the SAP Business Suite, as provided by SAP SE, Walldorf, Germany) or can be standalone computing products and/or functionalities. Upon activation within the applications, the functionalities can be used to generate the user interface provided via the input/output device 740. The user interface can be generated and presented to a user by the computing system 700 (e.g., on a computer screen monitor, etc.).

One or more aspects or features of the subject matter described herein can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system may include clients and servers. A client and server are generally remote from each other and typically interact through a communication network. The relationship of client and server arises by virtue of computer programs running on the respective computers and having a client-server relationship to each other.

These computer programs, which can also be referred to programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including, for example, acoustic, speech, or tactile input. Other possible input devices include, but are not limited to, touch screens or other touch-sensitive devices such as single or multi-point resistive or capacitive trackpads, voice recognition hardware and software, optical scanners, optical pointers, digital image capture devices and associated interpretation software, and the like.

In the descriptions above and in the claims, phrases such as "at least one of' or "one or more of" may occur followed by a conjunctive list of elements or features. The term "and/or" may also occur in a list of two or more elements or features. Unless otherwise implicitly or explicitly contradicted by the context in which it used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." Use of the term "based on," above and in the claims is intended to mean, "based at least in part on," such that an unrecited feature or element is also permissible.

The subject matter described herein can be embodied in systems, apparatus, methods, and/or articles depending on the desired configuration. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the implementations described above can be directed to various combinations and subcombinations of the disclosed features and/or combinations and subcombinations of several further features disclosed above. In addition, the logic flows depicted in the accompanying figures and/or described herein do not necessarily require the particular order shown, or sequential order, to achieve desirable results. Other implementations may be within the scope of the following claims.

## Claims

1. A computer-implemented method, comprising:
training one or more machine learning models to predict a decline in employee performance;
applying, to a data associated with an employee, the one or more trained machine learning models to predict a decline in a performance of the employee;
in response to the one or more trained machine learning models predicting the decline in the performance of the employee, determining one or more root causes of the predicted decline in the performance of the employee; and
generating, based at least on the one or more root causes, a report including a corrective action for the predicted decline in the performance of the employee.

2. The method of claim 1, wherein the one or more root causes are identified by applying an explainability algorithm to determine a top k quantity of variables contributing to an output of the one or more machine learning models.

3. The method of claim 2, wherein the explainability algorithm comprises a Shapley Additive Explanations (SHAP).

4. The method of any one of the preceding claims, wherein the one or more machine learning models include a convolutional neural network, a recurrent neural network, a regression model, an instance-based model, a regularization model, a decision tree, a random forest, a Bayesian model, a clustering model, an associative model, a deep learning model, a dimensionality reduction model, and/or an ensemble model.

5. The method of any one of the preceding claims, wherein the training of the one or more machine learning models include
training, at a first client device associated with the employee, a first local machine learning model, the local machine learning model being trained based at least on a first personal data associated with the employee, and
updating, based at least on a first parameter space of the trained first local machine learning model, a second parameter space of a global machine learning model deployed at a server.

6. The method of claim 5, wherein the training of the one or more machine learning models further include
updating, based at least on a third parameter space of a second local machine learning model, the second parameter space of the global machine learning model, the second machine learning model being deployed at a second client device of another employee, and the second machine learning model being trained based on a second personal data of the another employee, and
updating, based at least on the updated second parameter space of the global machine learning model, the first parameter space of the first local machine learning model and the third parameter space of the second local machine learning model.

7. The method of any one of the preceding claims, wherein the operations further comprise:
preprocessing at least a portion of the data associated with the employee, the preprocessing includes performing a sentiment analysis to determine a type of sentiment, a fatigue level, an alertness level, and/or an engagement level exhibited by the employee.

8. The method of any one of the preceding claims, wherein the data associated with the employee corresponds to one or more leading indicators of employee burnout.

9. The method of any one of the preceding claims, wherein the data associated with the employee includes a physical activity pattern, a heartbeat pattern, a meal pattern, and/or a sleep pattern of the employee.

10. The method of any one of the preceding claims, wherein the data associated with the employee includes a quantity of time spent interacting with different types of applications installed on an edge device of the employee.

11. The method of any one of the preceding claims, wherein the data associated with the employee includes a quantity of deadlines, an urgency of deadlines, and/or a delay in meeting deadlines.

12. The method of any one of the preceding claims, wherein the data associated with the employee includes one or more key events, personal milestones, professional milestones, holidays, seasonal constraints, and job role constraints.

13. A system, comprising:
at least one data processor; and
at least one memory storing instructions, which when executed by the at least one data processor, result in operations according to a method of any one of claims 1 to 12.

14. A non-transitory computer readable medium storing instructions, which when executed by at least one data processor, result in operations according to a method of any one of claims 1 to 12.
